# EUROPEAN PATENT APPLICATION

(11) **EP 4 245 332 A1**
(43) Date of publication of application: **20.09.2023**
(21) Application number: 22162353.1
(22) Date of filing: 15.03.2022
(51) Int. Cl.: A61M 5/142, A61M 5/168

(54) **INFUSION DELIVERY DEVICE**

(71) Applicant: F. Hoffmann-La Roche AG, 4070 Basel (CH); Roche Diabetes Care GmbH, 68305 Mannheim (DE)
(72) Inventor: NORRICK, Nicklas, 68305 Mannheim (DE); KATZ, Jakob, 68305 Mannheim (DE)
(74) Representative: Pfaffmann, Lucas

(57) **Abstract**

The present invention relates to an infusion delivery device comprising a reservoir that is configured for containing a liquid medicament, a dispensing member that is moveably arranged within the reservoir for dispensing liquid medicament out of the reservoir through an outlet of the reservoir, an actuation member that is configured for moving the dispensing member within the reservoir, and a controller that is configured for controlling the movement of the dispensing member within the reservoir by the actuation member, wherein the controller is further configured to determine an occlusion hindering the dispensing of liquid medicament out of the reservoir and that is configured to provide a control signal to the actuation member, upon determining an occlusion, to move the dispensing member in a reverse direction about a displacement distance. In order to prevent an overdosing or underdosing in case of an occlusion, the displacement distance is a model-based displacement distance.

## Description

The present invention relates to an infusion delivery device comprising a reservoir that is configured for containing a liquid medicament, a dispensing member that is moveably arranged within the reservoir for dispensing liquid medicament out of the reservoir through an outlet of the reservoir, an actuation member that is configured for moving the dispensing member within the reservoir, and a controller that is configured for controlling the movement of the dispensing member within the reservoir by the actuation member, wherein the controller is further configured to determine an occlusion hindering the dispensing of liquid medicament out of the reservoir and that is configured to provide a control signal to the actuation member, upon determining an occlusion, to move the dispensing member in a reverse direction about a displacement distance.

During use, a hitherto known infusion delivery device is in fluid communication with a patient's body, such that a liquid medicament that has been previously filled into the reservoir of the infusion delivery device can be administered through the outlet out of the reservoir into the patient's body. The liquid medicament is dispensed by moving the dispensing member within the reservoir by the actuation member, wherein the actuation member is controlled by the controller of the infusion delivery device. Accordingly, the controller controls the amount of liquid medicament that is administered into the patient's body by controlling the movement of the displacement member with the help of the actuation member.

Controlling of the amount of medicament to be infused into the patient's body is of particular importance as the amount of medicament is intrinsically linked to a specific or desired medical purpose. An overdosing or an underdosing of the liquid medicament, respectively, may have severe negative effects for the patient's health.

Sometimes administering of the liquid medicament is partially or fully hindered by an occlusion of the outlet. Until determination of the occlusion the dispensing member is further advanced by the actuation member thereby increasing the pressure within the reservoir. Upon determination of an occlusion the controller of a hitherto known infusion delivery device is configured to provide a control signal to the actuation member to move the dispensing member in a reverse direction about a displacement distance. Still, due to the unpredictability of the increase of pressure there is a risk that the displacement distance does not meet the demands to level the pressure within the reservoir. The pressure may not be sufficiently decreased to lower the risk of an unintended overdosing, once the occlusion is suddenly resolved. On the other hand, the displacement distance may be too large, such that a negative pressure is created within the reservoir. Liquid medicament that was intended to be administered to the patient may be drawn in a reverse direction, i.e. into the reservoir again, thereby resulting an unintended underdosing as well as an extraction of bodily fluids.

Accordingly, it is an object of the present invention to provide an infusion delivery device that avoids at least one of the problems associated with infusion delivery devices known from prior art. In particular, it is an object of the invention to provide an infusion delivery device that, upon determination of an occlusion, lower the risks of an overdosing and underdosing of liquid medicament administered to a patient.

At least one of the above objects is solved by the provision of the subject matter disclosed in the claims and throughout the specification.

According to a first aspect, the infusion delivery device comprises a reservoir that is configured for containing a liquid medicament, a dispensing member that is moveably arranged within the reservoir for dispensing liquid medicament out of the reservoir through an outlet of the reservoir, an actuation member that is configured for moving the dispensing member within the reservoir, and a controller that is configured for controlling the movement of the dispensing member within the reservoir by the actuation member, wherein the controller is further configured to determine an occlusion hindering the dispensing of liquid medicament out of the reservoir and that is configured to provide a control signal to the actuation member, upon determining an occlusion, to move the dispensing member in a reverse direction about a displacement distance, wherein the displacement distance is a model-based displacement distance.

An infusion delivery device, such as for example an insulin pump or a medicament pump, comprises a reservoir that is configured for holding a liquid medicament that during use of the infusion delivery device is administered into a patient's body. A dispensing member is moveably arranged within the reservoir, wherein the liquid medicament can be dispensed by the movement of the dispensing member. When being actuated by the actuation member, the dispensing member pushes the liquid medicament out of the reservoir through the outlet of the reservoir and further into the patient's body. Thus, by controlling the movement of the dispensing member the amount of liquid medicament that is dispensed out of the reservoir is also controlled. The movement of the dispensing member is controlled by controlling the actuation member by the controller.

The controller is further configured to determine an occlusion, i.e. an unplanned fully or partial closure that hinders a flow of liquid medicament through the outlet. In the meaning of the present invention, the term outlet encompasses an opening in the reservoir as well as any tubing or fluid path that is in fluid communication with the opening in the reservoir, including any cannula, needle or the like for conveying the liquid medicament from the reservoir into a patient's body, during use of the infusion delivery device.

For instance, the controller can be configured to determine an occlusion by monitoring at least one parameter indicative of the amount of torque or force or energy needed by the actuation member to advance the dispensing member within the reservoir for dispensing liquid medicament. Alternatively or in addition, the controller can be configured to receive a notification by a supplementary sensor or detector or device that an occlusion occurred.

The controller is further configured to provide a control signal to the actuation member, upon determining an occlusion, to move the dispensing member in a reverse direction about a displacement distance. In the meaning of the present invention, the reverse direction is a direction opposite to the direction needed to dispense liquid medicament out of reservoir through the outlet. By being moved in a reverse direction about the displacement distance the pressure within the reservoir that has been build up by the dispensing member's movement intended to dispense liquid medicament out of the reservoir through the at least partially clogged outlet is lowered.

However, in order not to move the dispensing member too far in the reverse direction, i.e. about a displacement distance shorter or longer than needed to level the pressure within the reservoir, the displacement distance is a model-based displacement distance. Accordingly, the control signal to the actuation member, upon determining an occlusion, to move the dispensing member in a reverse direction about a displacement distance is a control signal to the actuation member, upon determining an occlusion, to move the dispensing member in a reverse direction by a model-based displacement distance. Thereby creation of an unintended negative pressure within the reservoir is avoided that otherwise could re-draw liquid medicament into the reservoir that was intended to be administered into the patient's body.

According to an embodiment, the model-based displacement distance takes into account a parameter indicative of the current position of the dispensing member within the reservoir and/or a parameter indicative of the temperature of the reservoir and/or a parameter indicative of the temperature of the medicament within the reservoir. The current position of the dispensing member is indicative of the amount or volume of medicament, respectively, in the reservoir and thus also indicative of the fraction of the reservoir that is in contact with the medicament. The size of the contact area is indicative of the deformability of the reservoir as well as of the force exerted by the medicament on the reservoir by a given pressure within the reservoir.

By way of example, in an embodiment the model-based displacement distance is smaller in case the current position of the dispensing member within the reservoir is closer to the outlet than in case the current position of the dispensing member within the reservoir is more distant from the outlet. The accuracy of transferring a parameter indicative of the current position of the dispensing member into a model-based displacement distance can be increased or decreased by the resolution of underlying metric. Each position of the dispensing member can be assigned to a different model-based displacement distance. On the other hand, each position of the dispensing member within a predetermined range can be assigned to the same model-based displacement distance. For instance, a first model-based displacement distance can be used in case the dispensing member's current position is within a first fracture of the maximum displacement distance of the dispensing member next to the outlet of the reservoir. A second model-based displacement distance can be used in case the dispensing member's current position is within a second fracture of the maximum displacement distance following the first fracture of the maximum displacement distance. A third model-based displacement distance of the dispensing member can be used in case the next dispensing member's current position is within a third fracture of the maximum displacement distance following the second fracture facing away the first fracture. The first fracture, the second fracture, and the third fracture can be equal representing a third of the maximum displacement distance. Alternatively, the size and number of the fractures can be set based on the model for the reservoir and plunger system.

Likewise, the temperature of the reservoir as well as the temperature of the medicament within the reservoir influences the pressure that can be build-up by advancing the dispensing member further within the reservoir. A model-based displacement distance taking into account a parameter indicative of the actual position of the dispensing member within the reservoir and/or a parameter indicative of the temperature of the reservoir and/or a parameter indicative of the temperature of the liquid medicament within the reservoir is beneficial for moving the dispensing member within the reservoir about a displacement distance appropriate to level the pressure within the reservoir in light of the current state of the reservoir and the dispensing member's position within the reservoir.

According to an embodiment, the dispensing member is a plunger or a membrane. The plunger is moveably arranged within the reservoir by the actuation member. By moving the plunger in a direction towards the outlet of the reservoir, liquid medicament contained in the volume between the plunger and the outlet is pushed towards the outlet and through the outlet. The distance by which the plunger is advanced within the reservoir is indicative of the amount of liquid medicament to be dispensed out of the reservoir. The membrane is also moveably arranged within the reservoir, wherein here "moving" may also mean deforming, such that at least a part of the membrane is deflected towards the outlet for dispensing liquid medicament out of the reservoir. Likewise, a movement in the reverse direction corresponds to a deflection of at least part of the membrane in a direction away from the outlet. By way of example, the actuation member for moving the membrane can be a hydraulic medium exerting on the membrane.

According to an embodiment, the actuation member comprises a stepper motor, wherein the model-based displacement distance takes into account an average number of missed steps per train for the stepper motor and/or the motor current. A stepper motor converts electric current into rotation, wherein the rotation is further converted into a longitudinal displacement of the dispensing member within the reservoir by means of a gearbox. Each pulse of electricity turns the stepper motor one step. A train is a sequence of steps corresponding to the standard driving pattern for the stepper motor. However, the average number of missed steps, i.e. electricity pulses that do not turn the motor one step, is indicative of the counterforce exerted on the dispensing member. An occlusion results in a higher counterforce and thus increases the number of missed steps per drivetrain. Furthermore, some electric pulses do not turn the motor one step, but are used up for settling and initial loading of components of the reservoir-dispensing member system, e.g. gaskets, O-rings, fittings, couplings etc. Likewise, the electric motor current is indicative of the counterforce provided by the dispensing member movement. The average number of missed steps per train for the stepper motor and/or the motor current can be used for adapting the model-based displacement distance to level the pressure within the reservoir in the light of the actual state of the reservoir and the dispensing member's position within the reservoir.

In an embodiment, the actuation member of the infusion delivery device is a motor, wherein the displacement distance is additionally or alternatively varied based on the motor current.

In an embodiment, the model-based displacement distance takes into account a parameter indicative of the pressure exerted by the liquid medicament within the reservoir on the dispensing member. The pressure within the reservoir that is exerted on the dispensing member can be directly or indirectly determined by a sensor or a detector, such as for example a pressure sensor or a force sensor. Determination of the pressure that is exerted by the liquid medicament within the reservoir on the dispensing member can be used to determine the model-based displacement distance to level the pressure within the reservoir. Thus, a parameter indicative of the pressure exerted by the liquid medicament within the reservoir on the dispensing member can be taken into account when determining the model-based displacement distance for controlling the actuation member to move the dispensing member within the reservoir to level the pressure within the reservoir thereby preventing an overdosing or an underdosing, respectively.

According to an embodiment, the model-based displacement distance takes into account a parameter indicative of the compressibility of the liquid medicament within the reservoir and/or a parameter indicative of the compressibility of the reservoir. The compressibility of the liquid medicament and/or the compressibility of the reservoir influences the pressure that is built-up within the reservoir when moving the dispensing member within the reservoir. In particular, in case of an occlusion that hinders a flow of liquid medicament out of the reservoir. Taking into account a parameter indicative of the compressibility of the liquid medicament within the reservoir helps to adapt the model-based displacement distance to level the pressure within the reservoir, once an occlusion occurred.

In an embodiment, the model-based displacement distance is provided by a look-up table. Providing the model-based displacement distance reduces the processing power needed by the controller to determine the respective model-based displacement distance needed to level the pressure within the reservoir in the light of the respective state of the reservoir and dispensing member position. The model-based displacement distance can also be provided by a combination of a look-up table and an equation that takes into account at least one input parameter. For example, the input parameter can be a sensor reading or a detector reading.

The present invention further relates to an infusion delivery device comprising a reservoir that is configured for containing a liquid medicament, a dispensing member that is moveably arranged within the reservoir for dispensing liquid medicament out of the reservoir through an outlet of the reservoir, an actuation member that is configured for moving the dispensing member within the reservoir, and a controller that is configured for controlling the movement of the dispensing member within the reservoir by the actuation member,
wherein the controller is further configured to determine an occlusion hindering the dispensing of liquid medicament out of the reservoir and that is configured to provide a control signal to the actuation member, upon determining an occlusion, to move the dispensing member in a reverse direction about a displacement distance, wherein the displacement distance is varied based on at least one of:
- a parameter indicative of the current position of the dispensing member within the reservoir, and/or
- a parameter indicative of the temperature of the reservoir, and/or
- a parameter indicative of the temperature of the medicament within the reservoir, and/or
- a parameter indicative of the pressure exerted on the dispensing member by the liquid medicament within the reservoir, and/or
- a parameter indicative of the compressibility of the liquid medicament within the reservoir, and/or
- a parameter indicative of the compressibility of the reservoir.

In an embodiment, the actuation member of the infusion delivery device is a stepper motor, wherein the displacement distance is additionally or alternatively varied based on the average number of missed steps per train for the stepper motor and/or the motor current.

In an embodiment, the actuation member of the infusion delivery device is a motor, wherein the displacement distance is additionally or alternatively varied based on the motor current.

The present invention further relates to a method for controlling an infusion delivery device comprising a reservoir that is configured for containing a liquid medicament, a dispensing member that is moveably arranged within the reservoir for dispensing liquid medicament out of the reservoir through an outlet of the reservoir, an actuation member that is configured for controlling the movement of the dispensing member within the reservoir by the actuation member, wherein the method comprises the steps of
- determining by the controller an occlusion of the outlet hindering the dispensing of the liquid medicament out of the reservoir,
- providing a control signal by the controller to the actuation member, upon determining an occlusion, to move the dispensing member in a reverse direction by a displacement distance,
wherein the step of providing a control signal by the controller to the actuation member, upon determining an occlusion, to move the dispensing member in a reverse direction by a displacement distance includes determining a model-based displacement distance as the displacement distance.

The controller controls the actuation member by which the dispensing member is moved within the reservoir, either in a direction to administer liquid medicament out of the outlet into a patient's body, or in an opposite direction, i.e. a reverse direction. By providing a control signal to the actuation member, upon determining an occlusion, to move the dispensing member in a reverse direction by a model-based displacement distance, the controller helps to prevent an overdosing or underdosing of liquid medicament in case of an occlusion as well as an extraction of bodily fluids. The model-based displacement is varied based on one or more parameters indicative of the current state of the infusion delivery device, in particular the current state of the reservoir and dispensing member. The current state of the reservoir and dispensing member with regards to the pressure caused thereby within reservoir may be influenced by various parameters, such as for example a parameter indicative of the temperature of the reservoir, and/or a parameter indicative of the temperature of the reservoir, and/or a parameter indicative of the temperature of the medicament within the reservoir, and/or a parameter indicative of the pressure exerted on the dispensing member by the liquid medicament within the reservoir, and/or a parameter indicative of the compressibility of the liquid medicament within the reservoir and/or a parameter indicative of the compressibility of the reservoir. Accordingly, in an embodiment, the step of providing a control signal to the actuation member, upon determining an occlusion, to move the dispensing member in a reverse direction by a displacement distance includes determining the model-based displacement distance based one or more of
- a parameter indicative of the current position of the dispensing member within the reservoir, and/or
- a parameter indicative of the temperature of the reservoir, and/or
- a parameter indicative of the temperature of the medicament within the reservoir, and/or
- a parameter indicative of the pressure exerted on the dispensing member by the liquid medicament within the reservoir, and/or
- a parameter indicative of the compressibility of the liquid medicament within the reservoir, and/or
- a parameter indicative of the compressibility of the reservoir.

As previously mentioned, the actuation member of the infusion delivery device can be a motor or a stepper motor, such that in addition or alternatively, the current state of the reservoir and dispensing member with regards to the pressure caused thereby within the reservoir may be influenced by the average number of missed steps per drive for the stepper motor and/or the motor current. Accordingly, in an embodiment, the step of providing a control signal to the actuation member, upon determining an occlusion, to move the dispensing member in a reverse direction by a displacement distance includes determining the model-based displacement distance based one or more of an average number of missed steps per train for the stepper motor, and/or the motor current.

The model-based displacement distance can be based on one or more of the aforementioned parameters as it has been outlined before with regards to an infusion delivery device according to the present invention.

Insofar, as in the foregoing as well as in the following detailed description of embodiments and claims, reference is made to an infusion delivery device, the characteristics described thereof are applicable for the method for controlling an infusion delivery device, and vice versa.

Further advantages, features and technical effects of the present invention are apparent from the following description of embodiments and the accompanying figures.
- Figure 1: shows a schematic view of an insulin delivery device according to an embodiment of the present invention;
- Figure 2: shows a schematic flow chart of a method for controlling an infusion delivery device according to an embodiment of the present invention; and
- Figure 3: shows an exemplary pressure gradient when taking into account a model-based displacement distance.

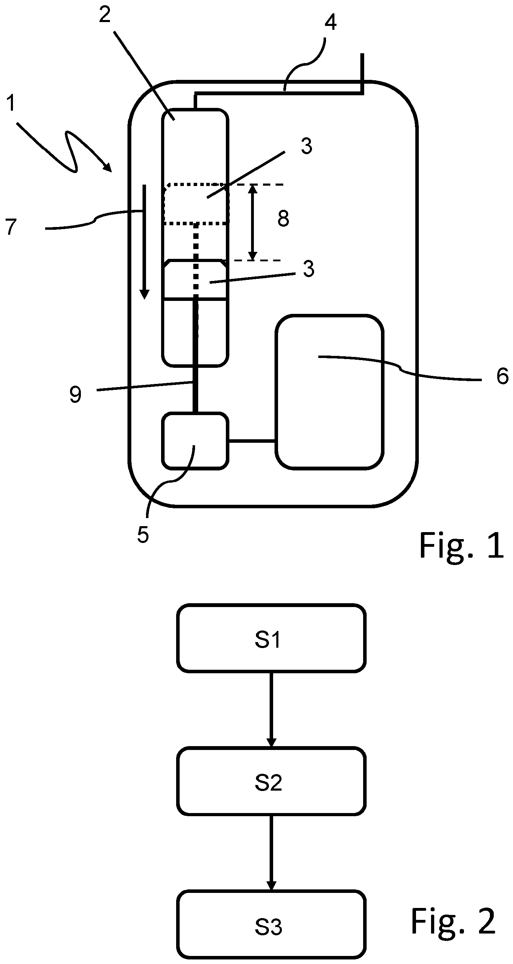

Figure 1 shows a schematic view of an insulin delivery device 1 according to an embodiment of the present invention. The insulin delivery device 1 comprises a reservoir 2 that is configured for holding a liquid medicament. Furthermore, the insulin delivery device 1 comprises a dispensing member 3 that is moveably arranged within the reservoir 2. Here, by way of example, the dispensing member 3 is a plunger. The dispensing member 3 is moved within the reservoir 2 by an actuation member 5 that is controlled by a controller 6. The controller 6 is configured to provide a control signal to the actuation member 5 to move the dispensing member 3. During use, upon moving the dispensing member 3 by the actuation member 5 in a direction towards the outlet 4 of reservoir for dispensing liquid medicament from the reservoir 2 to the patient's body via the outlet 4.

The controller 6 is further configured to determine an occlusion that may partially or fully hinder a flow of liquid medicament out of the reservoir 2 through the outlet 4 into a patient's body. In order not to cause an overdosing or underdosing in case of an occlusion, the controller is further configured to provide a control signal to the actuation member 5 to move the dispensing member 3 in a reverse direction 7 by a displacement distance. The displacement distance is a model-based displacement distance 8 that is determined by the controller 6 based on parameters indicative of the state of the reservoir 2 and dispensing member 3. Depending on the state of the reservoir 2 and dispensing member 3, such as for example the position of the dispensing member 3 within the reservoir 2, the model-based displacement distance 7 is varied so to level the pressure within the reservoir 2.

Figure 2 shows a schematic flowchart of a method for controlling an infusion delivery device 1 according to an embodiment of the present invention. The infusion delivery device 1 may be configured as shown in figure 1. Starting with steps S1 an occlusion is determined by the controller of the infusion delivery device 1 that hinders dispensing of liquid medicament out of the reservoir 2 of the infusion delivery device 1. Upon determining an occlusion, in step S2, the controller determines a model-based displacement distance 8 about which the dispensing member 3 is to be moved in a reverse direction 7 by the help of the actuation member 5 so to level the pressure within the reservoir 2 as desired. According to step S3 the model-based displacement distance 8 is provided to the actuation member 4 by the controller 6 as part of a control signal to move the dispensing member 3 in the reverse direction by the actuation member 5.

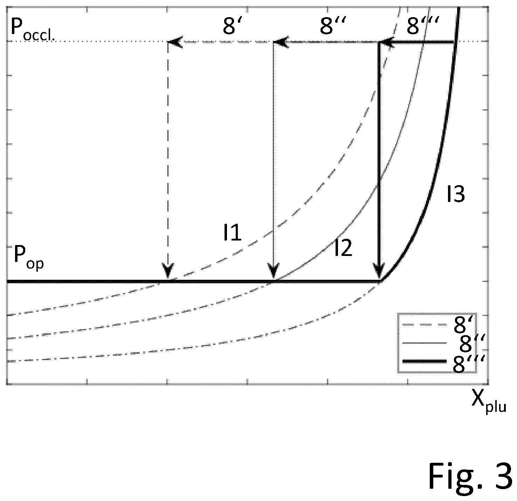

Figure 3 shows an exemplary pressure gradient that can be used for determining the model-based displacement distance 8 based on the current position Xₚₗᵤ of the dispensing member 3 within the reservoir 2. The model-based displacement distance 8 is used to level the pressure within the reservoir 2 from a pressure P_{occl} (pressure in case of occlusion) down to pressure Pₒₚ (pressure in case of normal operation). By way of example, the model-based displacement distance 8'" is smaller in case the dispensing member 3 is located within the last third I3 of the reservoir 2 closer to the outlet 4 than the model-based displacement distance 8" in case the dispensing member 3 is located within the second third I2 of the reservoir 2 closer to the outlet 4. The model-based displacement distance 8' is smaller in case the dispensing member 3 is located within the first third I1 of the reservoir 2 distant to the outlet 4 than the model-based displacement 8 in case the dispensing member 3 is located within the second third I2 of the reservoir 2 closer to the outlet 4.

### List of reference numbers

- 1: infusion delivery device
- 2: reservoir
- 3: dispensing member
- 4: outlet
- 5: actuation member
- 6: controller
- 7: reverse direction
- 8, 8', 8", 8‴: model-based displacement distance
- 9: dispensing member rod
- S1: determining occlusion
- S2: determining model-based displacement distance
- S3: providing control signal to actuation member
- I3: Interval next to the outlet
- I2: Interval next to the third interval I3
- I1: Interval next to the second interval 12, distant to the outlet
- P_{occl}.: Pressure within reservoir in case of occlusion
- Pₒₚ: Pressure within reservoir in case of normal operation
- Xₚₗᵤ: Position of plunger within reservoir

## Claims

1. An infusion delivery device (1) comprising
a reservoir (2) that is configured for containing a liquid medicament,
a dispensing member (3) that is moveably arranged within the reservoir for dispensing liquid medicament out of the reservoir through an outlet (4) of the reservoir,
an actuation member (5) that is configured for moving the dispensing member (3) within the reservoir (2), and
a controller (6) that is configured for controlling the movement of the dispensing member (3) within the reservoir (2) by the actuation member (5),
wherein the controller (6) is further configured to determine an occlusion hindering the dispensing of liquid medicament out of the reservoir (2) and that is configured to provide a control signal to the actuation member (5), upon determining an occlusion, to move the dispensing member (3) in a reverse direction (7) about a displacement distance (8), **characterized in that**
displacement distance is a model-based displacement distance.

2. Infusion delivery device (1) according to claim 1, **characterized in that** the model-based displacement distance (8) takes into account a parameter indicative of the current position of the dispensing member (3) within the reservoir (2) and/or a parameter indicative of the temperature of the reservoir (2) and/or a parameter indicative of the temperature of the liquid medicament within the reservoir (2).

3. Infusion delivery device (1) according to claim 1 or 2, **characterized in that** the actuation member (5) comprises a stepper motor, wherein the model-based displacement distance (8) takes into account an average number of missed steps per train for the stepper motor and/or the motor current.

4. Infusion delivery device (1) according to any one of claims 1 to 3, **characterized in that** the model-based displacement distance (8) takes into account a parameter indicative of the pressure exerted by the liquid medicament within the reservoir (2) on the dispensing member (3).

5. Infusion delivery device (1) according to any one of claims 1 to 4, **characterized in that** the model-based displacement distance (8) takes into account a parameter indicative of the compressibility of the liquid medicament within the reservoir (2).

6. Infusion delivery device (1) according to any one of claims 1 to 5, **characterized in that** the model-based displacement distance (8) is provided by a look-up table.

7. Method for controlling an infusion delivery device (1) comprising a reservoir (2) that is configured for containing a liquid medicament, a dispensing member (3) that is moveably arranged within the reservoir (2) for dispensing liquid medicament out of the reservoir (2) through an outlet (4) of the reservoir (2), an actuation member (5) that is configured for controlling the movement of the dispensing member (3) within the reservoir (2) by the actuation member (5), wherein the method comprises the steps of
• determining by a controller (6) an occlusion of the outlet hindering the dispensing of the liquid medicament out of the reservoir (2),
• providing a control signal by the controller (6) to the actuation member (5), upon determining an occlusion, to move the dispensing member (3) in a reverse direction (7) by a displacement distance,
**characterized in that** the step of providing a control signal by the controller (6) to the actuation member (5), upon determining an occlusion, to move the dispensing member (3) in a reverse direction (7) by a displacement distance includes determining a model-based displacement distance (8) as the displacement distance.

8. Method for controlling an infusion delivery device (1) according to claim 7, **characterized in that** the step of determining the model-based displacement distance is based on one or more of:
• a parameter indicative of the current position of the dispensing member (3) within the reservoir (2), and/or
• a parameter indicative of the temperature of the reservoir (2), and/or
• a parameter indicative of the temperature of the medicament within the reservoir (2), and/or
• a parameter indicative of the pressure exerted on the dispensing member by the liquid medicament within the reservoir (2), and/or
• a parameter indicative of the compressibility of the liquid medicament within the reservoir (2), and/or
• a parameter indicative of the compressibility of the reservoir (2).

9. Method for controlling an infusion delivery device (1) according to claim 7 or claim 8, **characterized in that** the actuation member (5) is a stepper motor, wherein the step of determining the model-based displacement distance is based on one or more of:
• an average number of missed steps per train for the stepper motor, and/or
• the motor current.
